# EUROPEAN PATENT APPLICATION

(11) **EP 4 497 469 A1**
(43) Date of publication of application: **29.01.2025**
(21) Application number: 23188109.5
(22) Date of filing: 27.07.2023
(51) Int. Cl.: A61N 5/06

(54) **WEARABLE PHOTOBIOMODULATION (PBM) DEVICE**

(71) Applicant: Seaborough Life Science B.V., 1098XG Amsterdam (NL)
(72) Inventor: BERENDS, Anne Claire, 3551 TJ Utrecht (NL); KRAMES, Michael, KENTFIELD, 94904 (US)
(74) Representative: Hoyng Rokh Monegier B.V.

(57) **Abstract**

A wearable electronic device (1), having a first radiation source (10) configured to emit a radiation beam (11) to a surface of skin of a user, the radiation beam having a peak emission wavelength between 610-1400nm; a driver circuit (20) configured to provide a driving current (15) to the radiation source (10) to control an irradiation intensity of the radiation beam on said surface; and an antenna (40) and/or a sensor circuit (30) having one or more sensors. The driver circuit (20) is configured to receive one or more inputs from the sensor circuit (30) and/or from the antenna (40) and is configured to control the first radiation source (10) on the basis of said one or more inputs.

## Description

### TECHNICAL FIELD

The invention relates to a wearable electronic device that delivers radiation sufficient to induce a photobiomodulation (PBM) effect.

### BACKGROUND ART

Photobiomodulation (PBM) involves irradiating a living organism at certain energy/power levels to induce biological or biochemical responses. The irradiation may be in the visible spectrum, such as red light, or in the non-visible spectrum, such as infrared (IR). There has been a significant amount of research about the medical benefits of employing PBM therapy to provide physical and psychological health benefits.

Most equipment that administers PBM radiation provides near-field excitation of the skin or treatment area (i.e. proximity devices) or uses very high power to irradiate the entire body. These specifications render the PBM effect exclusively in specialized medical or therapeutic devices that are often bulky and/or expensive. These factors greatly limit the availability of the health and wellness benefits of PBM within the general public.

In Applicant's PCT applications published as WO 2020/119965 and WO 2021/099642, herewith incorporated by reference in their entirety, a concept of incorporating PBM into general lighting has been proposed, using a lighting apparatus for emitting stable visible light and additionally emitting pulsed red or (near) infrared radiation to induce a PBM response. By driving a radiation source with a pulse instead of a continuous wave (or nearly continuous wave) signal, it is possible to boost the peak power during the pulses to achieve a PBM response. This allows the general lighting apparatus to emit a sufficient amount of power to enable a certain radiation intensity at a certain distance that may induce PBM responses. A sensor may be provided in such a lighting apparatus to turn on or off PBM radiation, depending on the presence and/or distance of the user.

It is desirable to implement PBM functionality in more non-specialist devices which are used in normal day-to-day activities, such as televisions, smartphones, tablets, laptops, etc. In this context, wearable electronic devices (such as smart watches) have a good potential in such implementation. The wearable electronic devices are typically used for extended hours during a user's daily life. For example, as long as the user wears a watch, the device could deliver PBM radiation to the user as needed. In addition, since these devices are positioned close to the user's skin, the delivered irradiation intensity of the red or (near) infrared radiation could be controlled accurately and reliably.

However, wearable electronic devices also have several limitations in delivering PBM radiation. Compared to known radiation devices, which project PBM radiation onto a relatively large surface such as the face of a user (typically larger than 200 cm²), the surface on which a wearable electronic device can radiate is relatively small. For example, even if a PBM radiation source is arranged across the back surface of a watch, the effective irradiation surface area is typically less than 25 cm². The exposure time could be increased to deliver a useful dose based on such a small surface area, but continuing to deliver PBM radiation over an extended period of time would result in the wearable electronic device quickly running out of (battery) power.

### SUMMARY OF THE INVENTION

It is an objective of the present invention to overcome the above-mentioned limitations and to provide a wearable electronic device which can efficiently induce a PBM response at the user.

The first aspect of the invention concerns a wearable electronic device, such as a watch, comprising: a first radiation source configured to emit a (first) radiation beam to a surface of skin of a user on which the wearable electronic device is worn, the radiation beam having a peak emission wavelength between 610-1400nm; a driver circuit configured to provide a driving current to the radiation source to control an irradiation intensity of the radiation beam on said surface; and an antenna and/or a sensor circuit having one or more sensors (e.g. detectors), wherein the driver circuit is configured to receive one or more inputs from the sensor circuit and/or from the antenna and is configured to control the first radiation source on the basis of said one or more inputs.

In an embodiment, said one or more inputs include an indication of a presence of a second radiation source. For example, the driver circuit may be configured to receive, from the sensor circuit or from the antenna, an indication of a presence of a second radiation source (e.g. sunlight and/or another radiation device), and wherein the driver circuit is configured to reduce said radiation intensity, on the basis of said indication.

According to this embodiment of the first aspect of the invention, the driver circuit is configured to receive an indication of a presence of a second radiation source (e.g. another device capable of PBM radiation, the sun, etc). The indication indicates the environment in which the wearable electronic device is worn, e.g. whether the user is expected to receive sufficient PBM radiation from a second radiation source. Such an indication may be implemented as one or more signals from the sensor circuit or from the antenna (including from both) which makes it possible to derive the presence of the second radiation source, preferably how strong the irradiance of the second radiation source is (e.g. the sunlight in sunny weather versus the sunlight in cloudy weather). This way, in an environment where the user is expected to receive natural or artificial irradiation capable of inducing a PBM response, the wearable electronic device reduces its irradiation intensity, for example by deactivating the first radiation source and/or adjusting its target delivered dose.

For example, the indication may indicate at least one of:
- the calculated irradiance from the second radiation source, determined at a regular interval while the device is in use;
- whether the wearable electronic device is worn in an outdoor environment or in an indoor environment;
- a weather condition in which the wearable electronic device is worn;
- whether the user is receiving a second radiation beam from a second radiation device.

The invention makes it possible for the user to continuously benefit from the PBM effect in an efficient manner, while the wearable electronic device can be used for that purpose during an extended period of time without running out of power quickly.

In an embodiment, the wearable electronic device is configured to periodically measure the irradiance of the second radiation source (e.g. using the antenna and or the one or more sensors) and adjust the first radiation source accordingly. This allows the present invention to be implemented in a simple manner. This wearable electronic may further be configured to adjust the first radiation source based on other indicators to optimize the system.

The wearable electronic device may comprise a housing, configured to accommodate at least one of the sensor circuit, the driver circuit and the antenna (on the surface of the housing and/or inside the housing), and possibly one or more other components such as a memory device. The housing may include a first surface having a display, and second surface substantially parallel to the first surface. The wearable electronic device may further comprise a band, configured to wrap around the skin of the user (e.g. wrist) so that the second surface of the housing is substantially attached to the skin of the user. The first radiation source may be disposed on the second surface of the housing. Additionality or alternatively, the first radiation source may be disposed on (a portion of) the band.

In an embodiment, the driver circuit is configured to switch between a first operation mode and a second operation mode, wherein, in the first operation mode (e.g. during normal operation) the radiation beam has a peak irradiation intensity above 0.1 mW/cm² at the surface (e.g. on a wrist of a user), and in the second operation mode the radiation beam has a peak irradiation intensity below 0.01 mW/cm² at said surface (e.g. switched off), wherein the driver circuit is configured to switch from the first operation mode to the second operation mode based on said indication.

In an embodiment, said one or more inputs (e.g. the indication) indicates whether the wearable electronic device is worn in an outdoor environment or in an indoor environment (e.g. based on the one or more sensors in the sensor circuit). Based on the indication, the wearable electronic device may be configured to only emit the radiation beam when the wearable electronic device is being worn by the user in an indoor environment (e.g. when the user is inside a building), and does not emit the radiation beam (or reduce the irradiation of the radiation beam or reduce the target dose) when the wearable electronic device is being worn by the user in an outdoor environment (e.g. when the user is not inside a building). In this way, in the outdoor environment, where the user is expected to receive sunlight as the second radiation source, unnecessary radiation from the wearable electronic device can be avoided, thereby saving its power.

In an embodiment, said outdoor environment is indicated based on one or more of: a sensed location, a sensed temperature, a sensed light condition, a sensed user breath rate, a sensed heartbeat rate, a sensed motion of the user, a strength of a received GPS signal, a strength of a received Wi-Fi signal, a user input. The outdoor environment may be indicated directly based on one or more of these factors, or indirectly based on one or more of other factors (e.g. the current activity of the user) derived from one or more of the factors mentioned above, or based on a combination of these two approaches.

In an embodiment, said one or more inputs (e.g. the indication) indicates a weather condition in which the wearable electronic device is worn. The weather condition may be indicated based on at least one of: the one or more sensors in the sensor circuit, a weather report, and a user input. The driver circuit may adjust the irradiation intensity of the (first) radiation beam based on said indication.

In an embodiment, said one or more inputs (e.g. the indication) indicates whether the user is receiving a second radiation beam from a second radiation device, based on a signal sent from the second radiation device or a control device to the wearable electronic device (e.g. to the antenna), and wherein the driver circuit is configured to adjust the irradiation intensity and/or duration (exposure time) and/or the target dose of the first irradiation beam in dependence on an irradiation intensity and/or duration and/or target dose of the second radiation beam.

The second radiation device may be a smartphone, a tablet, a laptop, a general lighting device, a USB powered accessory, or the like, which is equipped with its own radiation source for emitting the second radiation beam. The second radiation beam may have a peak emission wavelength between 610-1400nm having an irradiation intensity sufficient to induce PBM effect on the user. The wearable electronic device may be configured to communicate with the second radiation device (and/or a control device such as a smartphone), so that the driver circuit can adjust the irradiation intensity and/or duration and/or target dose of the (first) radiation beam based on the operation mode of the second radiation device.

For example, the driver circuit may be configured to decrease the irradiation intensity and/or duration and/or target dose of the (first) radiation beam when the second radiation beam has a higher radiation intensity (e.g. when the second radiation beam is switched on), and increase the irradiation intensity and/or duration and/or target dose of the (first) radiation beam when the second radiation beam has a lower intensity (e.g. when the second radiation beam is switched off).

In an embodiment, the wearable electronic device further comprises a dose control unit, wherein the driver circuit is configured to adjust the irradiation intensity, duration, and/or pulse condition of the radiation beam based on an input received from the dose control unit, such that a delivered dose of the radiation beam over a period of 4-8 hours is at least 140 J/cm² when the irradiation intensity is not reduced in accordance with said one or more inputs (e.g. the indication). This enables a sufficient total dose can be accumulated over time and across the limited area on said surface (e.g. in a range 10-60 cm²). The dose control unit may be configured such that a delivered total dose of the radiation beam over a period of 4-12 hours across said surface is in a range 1-10 kJ, or in a range 2-6 kJ, or in a range 3-7 kJ, or in a range 3-6 kJ, or in a range 4-8 kJ, or in a range 3-5 kJ, or in another range.

In an embodiment, the dose control unit is configured to receive an input from at least one of the sensor circuit and the antenna, to control the driver circuit on the basis of a combined dose of the wearable electronic device and the second radiation source. For example, the driver circuit may be configured to switch off the first radiation source, when a predetermined amount of the combined dose has been reached. The dose control unit may also be configured to adjust a target dose of the wearable electronic device in dependence on an accumulated dose delivered by the second radiation source. When the target dose is reached, the driver circuit may switch off the first radiation source.

In an embodiment, the dose control unit is configured such that the irradiation intensity of the (first) radiation beam is reduced (e.g. switched off) when a defined time has elapsed or when a defined dose has been accumulated, in dependence on said one or more inputs (e.g. the indication). In this way, the dose delivered from the second radiation source can be taken into account, and the dose received by the user from both the wearable electronic device and the second radiation source can be optimized together.

In an embodiment, the driver circuit is configured to switch between a first operation mode, a second operation mode, and a third operation mode, on the basis of said one or more inputs (e.g. the indication). The (first) radiation beam may have a first target dose in the first operation mode, a second target dose in the second operation mode, and a third target dose in the third operation mode, wherein the first target dose is lower than the second target dose, and the second target dose is lower than the third target dose. As an example, the first operation mode may be used when the user is in an outdoor environment with a sunny weather, the second operation mode may be used when the user is in an outdoor environment with a cloudy or rainy weather, and the third operation mode may be used when the user is in an indoor environment. The dose control unit may be used to ensure that these target doses are obtained.

In an embodiment, the wearable electronic device is a watch configured to emit the radiation beam onto a portion of a wrist of the user when the watch is worn by the user.

In an embodiment, the area of the surface of the skin of the user which receives the radiation beam is between 10cm² to 60cm².

In an embodiment, the wearable electronic device is configured to adjust an irradiation intensity of the radiation beam and/or exposure time of the radiation beam and/or target dose in dependence on one or more physical conditions (health) of the user, such as heart rate, maximum rate of oxygen consumption (VO₂ max), breathing characteristics, temperature, etc. These conditions may be determined based on the one or more sensors and/or based on user input. This enables the wearable electronic device to adjust the dose delivered to the user depending on such conditions. The wearable electronic device may also be configured to generate the radiation beam during a defined time during a day. This may for example allow PBM dosage to be delivered during the daytime period instead of night time period. Such delivery may also anticipate a time zone change and adjust the dosage delivery accordingly. Additionally or alternatively, the wearable electronic device may adjust the radiation beam during sport of a user (e.g. by communicating with a fitness app and/or fitness device, and/or based on user input). All in all, using facilities that are usually present in wearable electronic devices such as smart watches, the PBM dosage delivery could be optimized in various ways.

A second aspect of the invention concerns a system capable for providing a photobiomodulation (PBM) effect, comprising: the wearable electronic device according to the first aspect of the invention; and a second radiation device configured to operate in a first state and a second state, wherein, in the first state, the second radiation device emits a second radiation beam having a peak emission wavelength between 610-1400nm, and in the second state, the second radiation device does not emit the second radiation beam, and wherein the indication indicates whether the second radiation device operates in the first state or in the second state.

The second radiation device may be a smartphone, a tablet, a laptop, a general lighting device, a radiation device a car, train or other vehicles, etc. In particular, most wearable electronic devices such as smart watches are controlled using a smart phone. Smartphone users typically view their phones with a relatively long screentime at a relatively short and stable distance (e.g. 30 cm). In addition, most smartphones are equipped with facial detection / recognition functionality. Combining these features with the wearable electronic device according to the first aspect of the invention, an efficient PBM system can be utilized in which both devices work efficiently.

In an embodiment, the second radiation device is configured to detect a predetermined area of the body of a user, wherein the second radiation device is configured to operate in the first state and send a deactivation signal to the wearable electronic device as an indication that the second radiation source is present, when the predetermined area is detected within a defined distance; and wherein the second radiation device is configured to operate in the second state and send an activation signal to the wearable electronic device as an indication that the second radiation source is absent, when the predetermined area is not detected within a defined distance. In this way, the wearable electronic device emits PBM radiation when the second radiation device does not emit its PBM radiation and vise versa, based on a reliable (facial) detection / recognition, so that no radiation is wasted for either device.

In an embodiment, the predetermined area covers at least a portion of the face of the user.

A third aspect of the invention concerns a wearable electronic device, such as a watch, comprising: a first radiation source configured to emit a (first) radiation beam to a surface on skin a user on which the wearable electronic device is worn (e.g. a wrist), the radiation beam having a peak emission wavelength between 610-1400nm; a driver circuit configured to provide a driving current to the radiation source to control an irradiation intensity of the radiation beam on said surface; and a dose control unit, wherein the driver circuit is configured to adjust the irradiation intensity of the radiation beam based on an input received from the dose control unit, such that a delivered total dose of the radiation beam over a period of 4-8 hours is at least 1 kJ (at least when the irradiation intensity is not reduced in accordance with said one or more inputs such as the indication).

The wearable electronic device according to the third aspect of the invention may further comprise one or more elements described above in the context of the first aspect of the invention, and may be configured to interact with the second radiation device described above in the context of the second aspect of the invention.

A forth aspect of the invention concerns a wearable electronic device, comprising: a first radiation source configured to emit a (first) radiation beam to a body part of the user on which the wearable electronic device is worn, the irradiated body part of the user having a projected area smaller than 200 cm²; the radiation beam having a peak emission wavelength between 610-1400nm; a driver circuit configured to provide a driving current to the radiation source to control an irradiation intensity of the radiation beam on said body part; and a dose control unit, wherein the driver circuit is configured to adjust the irradiation intensity of the radiation beam based on an input received from the dose control unit.

A fifth aspect of the invention concerns an electronic device, comprising: a first radiation source configured to emit a (first) radiation beam to a body part of the user; the radiation beam having a peak emission wavelength between 610-1400nm; a driver circuit configured to provide a driving current to the radiation source to control an irradiation intensity of the radiation beam on said body part; and an antenna and/or a sensor circuit having one or more sensors (e.g. detectors); wherein the driver circuit is configured to receive, from the sensor circuit or from the antenna, an indication of a presence of a second radiation source (e.g. sunlight and/or another radiation device) and/or information on the physical condition of the user (e.g. via a wearable electronic device), and wherein the driver circuit is configured to reduce said radiation intensity, on the basis of said indication. The electronic device may be implemented as the second radiation device in the system according to the second aspect of the invention.

### BRIEF DESCRIPTION OF THE DRAWINGS

Embodiments will now be described, by way of example only, with reference to the accompanying schematic drawings in which corresponding reference symbols indicate corresponding parts, and in which:
Figs. 1A to 1C schematically show embodiments of the wearable electronic device 1 according to the present invention.
Fig. 2 shows block schematic of an embodiment of the wearable electronic device 1 according to the invention.
Fig. 3 shows an embodiment of the wearable electronic device 1 according to the present invention, in which the wearable electronic device 1 is configured to sense whether the user is in an outdoor environment or in an indoor environment.
Fig. 4 shows an embodiment of the wearable electronic device 1 according to the present invention, in which the wearable electronic device 1 is configured to operate in dependence on the weather condition.
Fig. 5 shows an embodiment of the wearable electronic device 1 according to a first aspect of the present invention, and a system capable for providing a photobiomodulation (PBM) effect according to a second aspect of the present invention. In this embodiment, the wearable electronic device 1 is configured to establish the presence of a second device 2 capable of emitting a second radiation beam 21.
Fig. 6 shows an embodiment of the wearable electronic device 1 according to the present invention, in which the wearable electronic device 1 comprises a dose control unit 50 to control the dose.

### DESCRIPTION OF ILLUSTRATIVE EMBODIMENTS

The following is a description of certain embodiments of the invention, given by way of example only and with reference to the drawings.

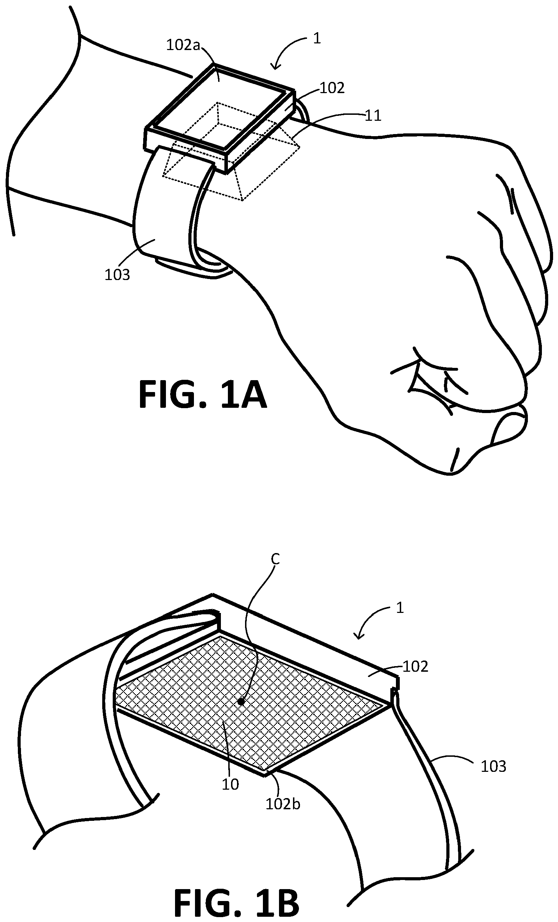

Fig. 1A shows a perspective view of an embodiment of a wearable electronic device 1 (such as a watch) according to the present invention.

The wearable electronic device 1 may comprise a housing 102, which contains one or more internal components such as one or more integrated circuit chips, circuit boards, display devices, batteries, memory devices, one or more sensors, one or more antennas, or other functional components. The wearable electronic device 1 is configured to emit the radiation beam 11 to a surface of the skin of a user. The wearable electronic device 1 is designed to be worn by the user over an extended time period during normal daily activities, such as a watch, article of clothing, piece of jewelry, etc.

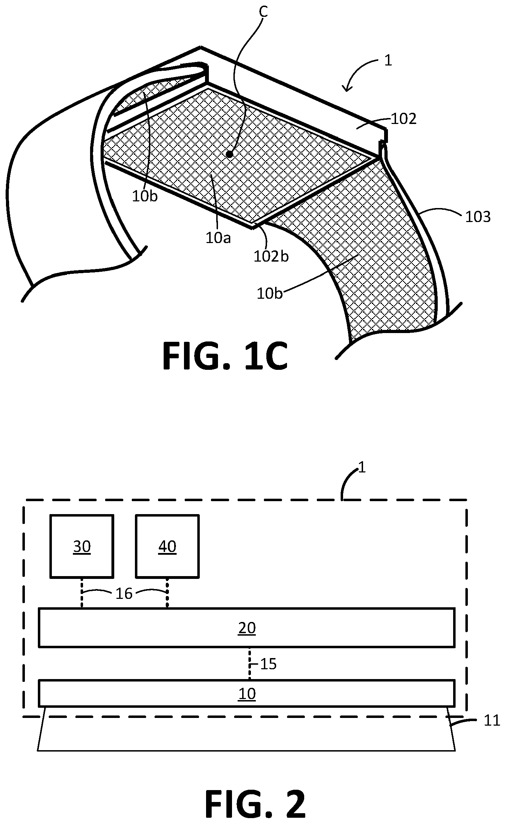

The housing 102 may include a first surface 102a (e.g. front surface) including a display and a second surface 102b (e.g. back surface) facing towards and in close proximity to the user's skin (e.g. at the user's wrist). The housing 102 may be implemented as any suitable structure that serves to enclose these internal components and may be directly or indirectly coupled to a band 103 to allow the housing 102 to be worn on the user's wrist. Although Fig. 1A shows a (smart)watch, other types of wearable electronic devices (e.g. smart bands, jewelry, clothing items) may also be used. Moreover, although Fig. 1A shows a generally rectangular structure of the wearable electronic device 1, the housing 102 may have any appropriate size or shape, such as round, hexagonal, square, or other shapes. Furthermore, although Fig. 1 shows that the radiation beam 11 is emitted from the back surface of the wearable electronic device 1, it may additionally or alternatively be emitted from another portion of the wearable electronic device 1, such as from the band 103.

Fig. 1B shows another perspective view of the embodiment shown in Fig. 1A. The wearable electronic device 1 comprises a first radiation source 10 configured to emit the radiation beam 11. For example, the first radiation source 10 may be disposed on the second surface (e.g. back surface) 102b of the wearable electronic device 1, as shown in Fig. 1B. Alternatively or additionally, the first radiation source 10 may also be disposed on another portion of the wearable electronic device (not shown), such as on a portion of the band 103.

The wearable electronic device 1 is preferably configured to emit the radiation beam 11 onto the skin of the user in the area substantially only underneath the wearable device, e.g. exposing the wrist of a user to the radiation beam 11 under a watch and/or watch band worn by the user, but not other portions of the user's body. The wearable electronic device 1 is preferably configured to hold the first radiation source in a substantially fixed position in close proximity to the skin of the user when the device is worn by the user, for example with a distance of 3 mm or less between the surface of wearable device in the location of the radiation source 10 and the skin of the user.

The radiation source 10 may comprise a plurality of LEDs and/or one or more edge-emitting laser diodes (EELDs) and/or vertical-cavity surface-emitting lasers (VCSELs), to emit the radiation beam. In the following description, the term "LEDs" is used for brevity, but should be understood to encompass LEDS, EELDs and/or VCSELs which may all be used for the first radiation source 10. The radiation beam has a peak emission wavelength between 610-1400nm. In an embodiment, the peak emission wavelength is in the (near) infrared ((N)IR) region, such as 700-1400 nm. In an embodiment this range may be 760-1400 nm or 800-1100 nm. Another option is the range 800-870 nm. The peak emission wavelength may also be with the spectrum of red light such as the range 610-700 nm. Radiation within these ranges is referred to herein as "PBM radiation". Preferably, substantially all of the radiation emitted by the radiation source 10 is within the range for the peak emission wavelength, for example 90% or 95% or 99% of the radiation falls within 610-1400 nm, 700-1400 nm, 760-1400 nm or 800-1100 nm. Irradiating the predetermined area (e.g. user's face) with radiation in these spectrums can induce beneficial PBM responses. Some embodiments may utilize devices emitting in different wavelength regimes simultaneously. The LEDs, EELDs, and/or VCSELs may comprise semiconductor light sources based on AlGaInP, InGaP, InGaAsP, InAlGaAsP, AlGaAs, GaAs, InGaAs, and related materials systems as known in the art.

Some embodiments may use optical components such as diffusers, lenses and/or or waveguides to distribute the red or (near) infrared light over the target surface in order to achieve an intended irradiance level and/or dose in an efficient and cost-effective manner.

Fig. 1C shows a variation of the embodiment of Fig. 1A in which the first radiation source 10 comprises a plurality of LEDs, EELDs and/or VCSELs arranged in a first portion 10a on the second surface 102b and a second portion 10b on a surface (e.g. back surface) of both portions of the band 103 of the wearable electronic device 1.

The second portion 10b may comprise a plurality of low-power LEDs disposed over a large portion of the band 103 of an electronic wristwatch, so they are facing towards and in close proximity to the user's skin when the wristwatch is worn. This may be implemented, for example, by incorporating the LEDs in a flexible printed circuit board (PCB) incorporated into or attached to the band 103, although the LEDs may be mounted by other means. For example, a suitable LED is product no. QBLP601-IR3 from the QT-Brightek Chip LED Series. These LEDs are small (1.6 x 0.8 mm²) and deliver about 3 mW of radiation at 850 nm upon driving with a 20 mA current (and 1.4 V forward voltage).

The area available on the surface 102b for arranging the LEDs of the first radiation source 10, and thus the area of skin irradiated, may typically vary from 1 cm² for a small watch to 25 cm² for a large watch. This area greatly increases when both the surface 102b and band 103 is available for arranging the LEDs, and may typically vary from 10 cm² for a small watch and narrow band, to 60 cm² for a large watch with wide band. The LEDs may be laid out in various series/parallel string combinations to present a suitable driving voltage for a DC-DC driver circuit powered by the watch battery for driving the LEDs.

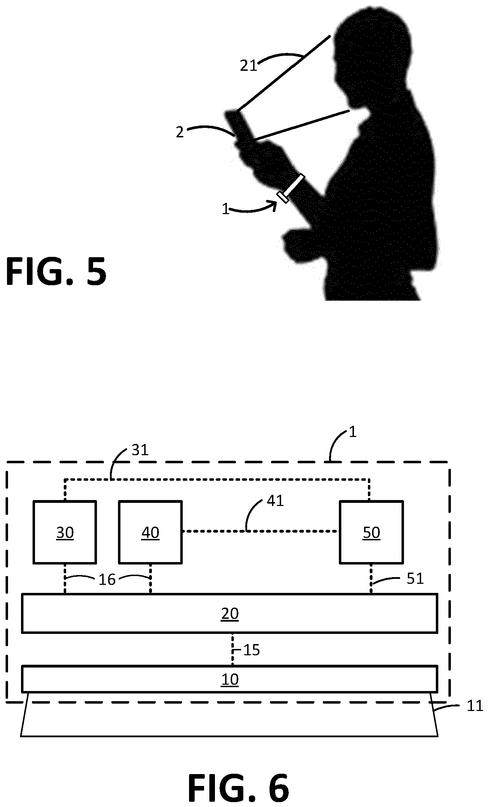

The driver circuit 20 may be configured to switch between a first operation mode and a second operation mode, wherein, in the first operation mode (e.g. during normal operation) the radiation beam has a peak irradiation intensity above 0.1 mW/cm² at the surface on a wrist of a user, and in the second operation mode the radiation beam has a peak irradiation intensity below 0.01 mW/cm² at said surface, wherein the driver circuit is configured to switch from the first operation mode to the second operation mode based on a control indication, such as the indication 16 discussed below. The irradiation intensity on the surface of the wrist of the user may be generally homogeneous or may vary to some extent across the surface. In the latter case, the particular values of the irradiation intensity may be measured at the center of the radiation beam 11, e.g., at a center point C of the back surface 102b of the wearable electronic device 1 as shown in Fig. 1B. Similarly, an accumulated dose (see below with reference to Fig. 6) may be derived in the same manner.

Fig. 2 shows a block schematic of an embodiment of the wearable electronic device 1 according to the invention. In the embodiment shown, the wearable electronic device 1 includes a radiation source 10, a driver circuit 20 configured to provide a driving current 15 (which may be pulsed or a continuous wave) to the radiation source 10 to control an irradiation intensity of the radiation beam on the surface of the user's skin; a sensor circuit 30 having one or more sensors; and an antenna 40. One or more of these components may be contained in the housing 102, in the band 103, or elsewhere in the wearable electronic device.

The sensor circuit 30 comprises one or more sensors, e.g. a motion sensor such as an accelerometer or velocity sensor, gyroscope, pulse (heart rate) sensor, breathing rate sensor, blood oxygen sensor, capacitive sensor, electromagnetic field sensor, light sensor, IR or NIR radiation sensor, image sensor, pressure or force sensor, touch sensor, vibration sensor, thermal sensor or temperature sensor, orientation sensor, location sensor (e.g., global positioning system (GPS) device), communication device (e.g., wireless communication device such as a Wi-Fi chip), resistive sensor, and the like. Wearable electronic devices such as smartwatches typically use such sensors to influence its operation. Utilizing such sensors, the invention enables the wearable electronic device 1 to control the radiation beam 11 in accordance with the surrounding environment.

As shown in Fig. 2, the driver circuit 20 is configured to receive an indication 16 from the sensor circuit 30 and/or from the antenna 40. On the basis of the indication, the driver circuit 20 adjusts the irradiation intensity or duty-factor of the radiation beam 11 on the wrist of the user, for example by adjusting the driving current 15, such as adjusting an amplitude and/or a pulse width and/or frequency of the driving current 15. The indication 16 may indicate the environment in which the wearable electronic device is worn, including received irradiance levels originating from sources other than the first radiation source 10. That is to say, whether the user is expected to receive sufficient PBM radiation from a second radiation source or at least a portion of a desired amount of PBM radiation from a second radiation source.

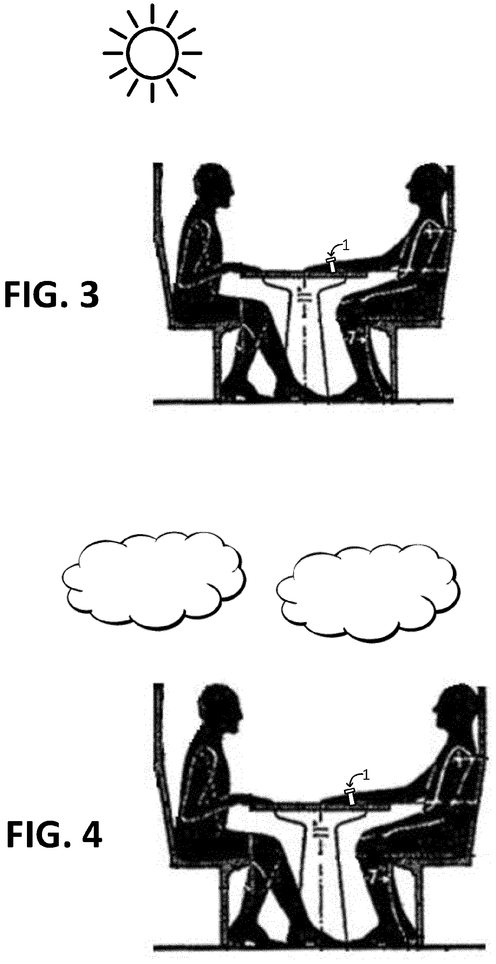

Fig. 3 shows an embodiment in which the wearable electronic device 1 is configured to sense whether the user is in an outdoor environment or in an indoor environment. An outdoor environment may be detected based on many factors, such as a sensed location, a sensed temperature, a sensed light condition, user activity based on sensed motion, sensed user breath rate or heartbeat rate and/or user input, strength of GPS signals, strength of Wi-Fi signals, etc., or a combination of one or more of these factors.

In this embodiment, the wearable electronic device 1 is configured to reduce the irradiation intensity of the radiation beam 11 when the wearable electronic device 1 is worn in the outdoor environment, where the user is expected to receive sunlight as a natural PBM source. For example, the first radiation source 10 may be switched off when the outdoor environment is detected. By reducing the irradiation intensity of the radiation beam 11 when it is not needed, battery life can be saved for the time it becomes needed, such as when the user enters a building or when the weather becomes worse (as discussed below).

Fig. 4 shows an embodiment in which the driver circuit 12 is configured to adjust the irradiation intensity of the radiation beam 11 in dependence on a detection of the environment and the weather. The weather condition may be sensed by the one or more sensors, based on a weather report retrieved from the internet via a GPS or wireless communication device, based on user input, or a combination of these.

In sunny weather where there is a strong presence of sunlight, the first radiation source may be switched off. In contrast, in cloudy weather as shown in Fig. 4 (or in rainy weather) where the presence of the sunlight is weaker, the first radiation source 11 may be switched on but emit the radiation beam 11 at a lowered irradiation intensity, or for a shorter time, or with an adapted pulse mode, or with a reduced duty-factor, to compensate for the reduction in sunlight. In the case where the user is inside a building, the radiation source 10 may produce the radiation beam 11 at the normal irradiation intensity. In other words, based on the indication 16, the driver circuit 12 may operate to control the radiation beam 11 with various different irradiation intensities, pulse modes, or ON time (including switching it off), to use just enough power to keep offering a desired level of PBM effect to the user.

### A system in which plural PBM devices work together

Fig. 5 shows an embodiment of the wearable electronic device 1 according to the present invention, which is configured to establish the presence of a second device 2 capable of emitting a second radiation beam 21. The second device 2 may be a smartphone, as shown in the figure.

Most users of smart watches usually also have a smartphone, and most smart watches are capable of communicating with a smartphone, which itself is suitable equipment for providing PBM effect, e.g. via a second radiation beam 21. Many users typically have a relatively long screentime with such devices and keep a relatively short and stable distance with the screen (e.g., 30 cm). Moreover, many smartphones are equipped with facial detection / recognition functionality, which allows the smartphones to emit the second radiation beam 21 upon detection of the user.

The embodiment of Fig. 5 is based on an insight that a very efficient system can be implemented by combining the "smartness" of a smartphone and a wearable electronic device 1 such as a smart watch. In this embodiment, the indication 16 is a signal received via the antenna 40 of the wearable electronic device 1, indicating whether the second device 2 is currently radiating the second radiation beam 21. This may be implemented using the facial detection / recognition functionality of the smart phone. For example, when the smartphone detects the user's face within an effective range (e.g., in a range 20 - 50 cm, such as 30 cm), the smartphone may begin to emit the second radiation beam 21 and send a deactivation signal to the wearable device 1. Upon receiving the deactivation signal on its antenna 40, the wearable electronic device 1 may then deactivate the radiation source 10. Conversely, when the smartphone detects the user's face is not within an effective range, the smartphone may stop emitting the second radiation beam 21 and send an activation signal to the wearable device 1. Upon receiving the activation signal on its antenna 40, the wearable electronic device 1 may then activate the radiation source 10. In this way, neither the wearable electronic device 1 nor the smartphone 2 wastes any power on unnecessary PBM radiation, while the user can continuously benefit from PBM effect.

The second device 2 may also be any device that can be equipped with an appropriate red or (near)infrared radiation source, such as a laptop, a tablet, a general lighting device as described in WO 2020/119965 and WO 2021/099642, etc. In these embodiments, the operation state of the second device 2 may similarly be indicated to the wearable electronic 1 based on communication between these devices, or may be indicated by another control device such as a smart phone.

The second device 2 may include its own radiation source (not shown), which may include a plurality of LEDs and/or one or more EELDs or VCSELs (including VCSEL arrays), to emit the second radiation beam 21. Similar to the (first) radiation beam 11, the second radiation beam 21 may have a peak emission wavelength between 610-1400nm. In an embodiment, the peak emission wavelength of the second radiation beam 21 is in the (near) infrared ((N)IR) region, such as 700-1400 nm. In an embodiment this range may be 760-1400 nm or 800-1100 nm. Another option is the range 800-870 nm. The peak emission wavelength may also be with the spectrum of red light such as the range 610-700 nm. Preferably, substantially all of the radiation of the second radiation beam 21 is within the range for the peak emission wavelength, for example 90% or 95% or 99% of the radiation falling within the range. Irradiating the predetermined area (e.g., the user's face) with radiation in these spectrums can induce beneficial PBM responses. Some embodiments may utilize devices emitting in different wavelength regimes simultaneously.

### Dose control

Fig. 6 shows an embodiment in which the wearable electronic device 1 further comprises a dose control unit 50 to control the dose of PBM radiation received by the user.

The dose control unit 50 may comprise a timer and/or logic to calculate an accumulated dose and/or a dose rate. Dose control unit 50 may be implemented as hardware logic circuits or a combination of hardware and software or firmware, and may be combined with other circuits performing other functions of the wearable electronic device 1.

Dose is typically expressed in the unit of the accumulated energy per square centimeter, i.e., J/cm², or as a total dose value expressed as energy in kJ. It is generally believed that once the accumulated dose has exceeded a certain optimum level, the overall PBM effect will begin to decrease, and continuing to accumulate dose might even become detrimental to the body. It is currently under debate what precisely the optimum dose level should be. This discussion is hindered by often poorly defined irradiated surface areas, changes in wavelength and other light characteristics, and the variety of outcome parameters that is being monitored. Nevertheless, the inventors have determined that an accumulated dose of a few to tens of J/cm² (e.g. 5-50 J/cm²) can have positive effects (see M.C. Gimenez et al, "Effects of Near-Infrared Light on Well-Being and Health in Human Subjects with Mild Sleep-Related Complaints: A Double-Blind, Randomized, Placebo-Controlled Study," Biology 2023, 12(1), 60).

The calculation of accumulated dose by dose control unit 50 preferably takes into account the PBM radiation received by the user from the radiation source 10 of the wearable device 1, and also any PBM radiation received from one or more other sources, such as the sun and/or a second device 2 which emits PBM radiation. The accumulated dose received from the radiation source 10 may be calculated based on the skin area of the user exposed to the PBM radiation from the radiation source 10, the exposure time (i.e. time period during which the wearable device is worn by the user and the radiation source 10 is emitting PBM radiation), and the duty cycle and irradiance (mW/cm²) of the LEDs of the radiation source 10. In a simple implementation, the accumulated dose from the radiation source 10 may be calculated based on the time period during which the wearable device is worn by the user and the radiation source 10 is switched on.

The accumulated dose received from the other PBM radiation sources may be calculated based on an estimate or a measurement of the dose received from the other sources. An estimate of the received dose, for example, may be based on indication 31 and/or 41 regarding the detected environment received from sensor circuit 30 and/or antenna 40 (as discussed above) and elapsed time of exposure to that environment (e.g. as determined by a timer circuit in the dose control unit 50). In a simple implementation, the accumulated dose from other PBM radiation sources may be calculated based on the time period in which the wearable device 1 is worn by the user in an outside environment. A measurement of the received dose, for example, may be based on indication 31 from sensor circuit 30 (e.g. from an IR or NIR radiation sensor which measures irradiance of received PBM radiation), and the time period over which the radiation is received (e.g. as determined by a timer circuit in the dose control unit 50).

Dose control unit 50 may provide an indication 51 which is used to control the radiation source 10. The indication 51 may be a signal which simply indicates when a predetermined dose has been reached, or a signal having a variable value indicating a current accumulated dose, a signal indicating a rate at which the dose is accumulating, a control signal to control an intensity of radiation emitted by radiation source 10, or some other type of indication or control signal related to the dose. The driver circuit 20 may be configured to receive the indication 51 and in response, stop providing driving current 15 to radiation source 10, or alter the driving current 15 to alter the intensity of the radiation beam 11. For example, the indication 51 may control the driver circuit 20 to reduce the driving current 15 when the wearable device 1 is worn by the user in an outside environment, and may control the driver circuit 20 to stop the driving current 15 when a predetermined accumulated dose is calculated by dose control unit 50.

In this way, the contribution of any PBM radiation received from sources other than the wearable device 1 can be taken into account to control the radiation source 10 of the wearable device. This enables the wearable device 1 to save power by reducing the radiation emitted by radiation source 1 when it is not needed.

The wearable device 1 may adjust the dose delivered to the user in dependence on the physical condition of the user. For example, if the wearable device 1 senses that the user's condition is not optimal (e.g. based on resting heart rate, VO₂ max, breathing characteristics, or otherwise) it can deliver a higher dose to aid the recovery of the user. The wearable device 1 may also adjust the dose it delivers depending on other user characteristics like age, gender, pregnancy, nutrition, hormone cycles, menstruation cycle, or genes. The wearable PBM device may also communicate with other devices (e.g. smartphone or laptop) to instruct those devices to adjust the PBM radiations of those devices, to (temporarily) increase the daily dose.

In another embodiment, the wearable device 1 is configured (e.g. programmed) such that the optimal PBM dose is delivered at, before, or after a specific time of day. This may be favorable to enhance the circadian rhythm of the user, or to not disturb it, or to adjust it. The preferred time of day for PBM dosage delivery may be adapted when the device senses a change of time-zone, or based on a user input. In addition, it may change the preferred time of day of PBM dosage delivery before a time-zone change takes place, to aid the user adjust to the new time-zone in advance.

The ideal time of day for PBM dosage delivery may also be adapted to the lifestyle of the user, e.g. depending on shiftwork and the associated (social) jetlag and/or desire to perform at night time and not fall asleep. In addition, the ideal time of day for dosage delivery may be adapted to the personal biology of the user such as age, gender, pregnancy, nutrition, hormone cycles, menstruation cycle, or genes.

In another embodiment, a PBM device may communicate with fitness apps and/or fitness devices or respond to a user input, to contribute to optimal sports performance of the user. For example, the timing of the dose delivery may be adjusted to the exercise schedule of the user. For example, depending on the type of exercise, (aerobic or anaerobic, training or race day, etc.) the dose can be delivered before the sport activity for preparation, or during for performance boost, or afterwards to aid recovery.

In the context of using a wearable electronic device 1 to induce PBM effect, where the surface area affected by the radiation beam is very limited, the inventors have come to the realization that the accumulated dose across the entire irradiated area could be the actual decisive factor. Such accumulated dose may be a few kJ. To achieve this, a much higher dose (per square centimeter) than typical configurations is needed, i.e., 50 to 140 kJ/cm² or more. Table 1 shows several embodiments, in which an accumulated total dose of about 4 kJ across the irradiated area on the wrist of the user over an 8-hour exposure is achieved:

**Table 1**

| Exposed skin area on the wrist (cm²) | Exposure time (hour) | Duty cycle (%) | Peak irradiance (mW/cm²) | Dose (J/cm²) | Total dose (kJ) |
|---|---|---|---|---|---|
| 13.6 | 8 | 100 | 10 | 288 | 3.9 |
| 23.9 | 8 | 100 | 6 | 173 | 4.1 |
| 1 | 8 | 100 | 140 | 4032 | 4.0 |

In one example, a large watch and band has an irradiated area of 60 cm², with one LED per 3 square cm for a total of 20 LEDs and delivering a total radiation of 60 mW, for an average irradiance of 1 mW/cm². If the LEDs are operated continuously (100% duty factor) for 8 hours, an average dose of 28.8 J/cm² is achieved, for a total energy delivery of 1.73 kJ. This is about 43% of the total energy of a dose delivered at 6.5 J/cm² over a typical human face, neck and hands, a dose which is known to provide positive benefits to humans and discovered by the inventors. The total power supplied to the LEDs in this example is about 0.6 W, and total energy consumed over 8 hrs is about 4.8 W-h. In order to supply this power to the LEDs, the battery in the watch must have a capacity larger than this, e.g., about 5 to 7 W-h total, to also operate the watch functions.

The above example highlights the importance of the efficiency of the LEDs, the improvement of which will serve to reduce power consumption and extend battery life. In another example, a single high-power, high-efficiency IR LED is positioned at the back face of an electronic wristwatch, so it is facing the user's skin. This may be done by incorporating the LED in a PCB facing towards the back face of the watch, although the LED may be mounted by other means. A suitable LED for this embodiment is product no. SFH 4170S from the OSRAM OSLON^{®} P1616 series. This LED is small (1.6 x 1.6 mm²) and delivers about 70 mW of radiation at 850 nm upon driving with a 70 mA current (and 2.7 V forward voltage). Assuming an optical system (lens and/or diffusor) is attached to the LED so the emitted radiation covers an area on the skin of 5 x 5 mm², this single LED will deliver an average irradiance of about 280 mW/cm², which is below the maximum exposure limit of human skin for 850 nm irradiation which is about 400 mW/cm² (see M.H. Smith et al., "Safe delivery of optical power from space," Optics Express Vol. 8 No. 10, p.537, 7 May 2001). If the LED is operated continuously (100% duty factor) for 8 hours, a dose of 8.1 kJ/cm² is achieved, for a total energy delivery of 2 kJ. This is about 50% of the total energy of a dose delivered at 6.5 J/cm² over a typical human face, neck and hands to provide positive benefits. The total power supplied to the LED is about 0.19 W, and total energy consumed over 8 hrs is about 1.5 W-h. In order to supply power to the LEDs, the battery in the watch must have a capacity larger than this, about 2.5 to 3.5 W-h total, to also operate the watch functions.

Of course, especially in the case wherein the wearable electronic device 1 is contributing a dose of PBM radiation in combination with one or more other radiation sources (such as other devices or the sun), a lower total energy delivery is needed for the radiation source 10 which conserves energy and battery life of the wearable electronic device 1. In the second example above, for example, the total delivered energy may be turned down to 20% of the total energy of a dose delivered at 6.5 J/cm² over a typical human face, neck and hands. This may be accomplished by turning down the LED current (e.g., to 22.5 mA) and allows the possibility to use a smaller, lower cost, LED chip. In this case, the total energy consumed over 8 hrs is about 0.5 W-h which is within the battery capacity of many electronic wristwatches available commercially today. Of course, over time, battery capacities will improve and allow for more options in including IR LEDs or laser diodes for wearable PBM applications as anticipated by this invention.

As described above, the wearable electronic device 1 may take into account PBM effect delivered by a second radiation source (the sun, a smartphone, etc). The dose control unit 50 may be configured to control the radiation source 10 such that a combined dose is delivered from the wearable electronic device 1 and the second radiation source. For example, the dose control unit 50 may be configured such that the irradiation intensity of the radiation beam 11 is switched off or reduced when a defined time has elapsed or when a defined dose has been accumulated, in dependence on the indication. In this way, the dose delivered from the second radiation source can be taken into account, and the dose received by the user from both the wearable electronic device and the second radiation source can be optimized together.

Certain embodiments described above involve logic operations or calculations. These may be implemented using software (e.g., code embodied on a machine-readable medium or in a transmission signal), hardware, or a combination of these. In software implementation, one or more processors may be used. A hardware implementation may involve the use of dedicated circuitry or logic that is configured to perform certain operations. For example, a hardware module may be a programmable logic device such as a field programmable gate array (FPGA) or an ASIC. It will be appreciated that the decision to implement using software, hardware, or combination of these, may be driven by cost and time considerations.

The descriptions above are intended to be illustrative, not limiting. It will be apparent to the person skilled in the art that alternative and equivalent embodiments of the invention can be conceived and reduced to practice, without departing from the scope of the claims set out below.

## Claims

1. A wearable electronic device (1), comprising:
a first radiation source (10) configured to emit a radiation beam (11) to a surface of skin of a user on which the wearable electronic device (1) is worn, the radiation beam having a peak emission wavelength between 610-1400nm;
a driver circuit (20) configured to provide a driving current (15) to the radiation source (10) to control an irradiation intensity of the radiation beam on said surface; and
an antenna (40) and/or a sensor circuit (30) having one or more sensors;
wherein the driver circuit (20) is configured to receive one or more inputs from the sensor circuit (30) or from the antenna (40) and is configured to control the first radiation source (10) on the basis of said one or more inputs.

2. The wearable electronic device (1) according to claim 1, wherein the driver circuit (20) is configured to receive, from the sensor circuit (30) or from the antenna (40), an indication (16) of a presence of a second radiation source, and
wherein the driver circuit (20) is configured to reduce said radiation intensity, on the basis of said indication (16).

3. The wearable electronic device (1) according to claim 1 or 2, wherein the driver circuit (20) is configured to switch between a first operation mode and a second operation mode, wherein, in the first operation mode the radiation beam has a peak irradiation intensity above 0.1 mW/cm² at the surface, and in the second operation mode the radiation beam has a peak irradiation intensity below 0.01 mW/cm² at said surface, wherein the driver circuit is configured to switch from the first operation mode to the second operation mode based on said one or more inputs.

4. The wearable electronic device (1) according to any of the preceding claims, wherein said one or more inputs indicates whether the wearable electronic device (1) is worn in an outdoor environment or in an indoor environment, wherein said outdoor environment is preferably indicated based on one or more of: a sensed location, a sensed temperature, a sensed light condition, a sensed user breath rate, a sensed heartbeat rate, a sensed motion of the user, a strength of a received GPS signal, a strength of a received Wi-Fi signal, a user input.

5. The wearable electronic device (1) according to any of the preceding claims, wherein said one or more inputs indicates a weather condition in which the wearable electronic device (1) is worn.

6. The wearable electronic device (1) according to any of the preceding claims, wherein said radiation beam is a first radiation beam, and said one or more inputs indicates whether the user is receiving a second radiation beam (21) from a second radiation device (2), based on a signal sent from the second radiation device (2) or a control device to the wearable electronic device (1), and wherein the driver circuit is configured to adjust the radiation intensity of the first irradiation beam in dependence on an irradiation intensity of the second radiation beam (21).

7. The wearable electronic device (1) according to any of the preceding claims, further comprising a dose control unit (50), wherein the driver circuit (20) is configured to adjust the irradiation intensity of the radiation beam (11) based on an input (51) received from the dose control unit (50), such that a delivered dose of the radiation beam (11) over a period of 4-8 hours is at least 140 J/cm² when the irradiation intensity is not reduced in accordance with said one or more inputs.

8. The wearable electronic device (1) according to claim 7, wherein the dose control unit (50) is configured to receive an input from at least one of the sensor circuit (30) and the antenna (40), to control the driver circuit (20) on the basis of a combined dose of the wearable electronic device (1) and the second radiation source.

9. The wearable electronic device (1) according to claim 7 or 8, wherein the dose control unit (50) is configured such that the irradiation intensity of the radiation beam (11) is reduced when a defined time has elapsed or when a defined dose has been accumulated, in dependence on said one or more inputs.

10. The wearable electronic device (1) according to any of the preceding claims, wherein the driver circuit (20) is configured to switch between a first operation mode, a second operation mode, and a third operation mode, on the basis of said one or more inputs, wherein the radiation beam (11) has a first target dose in the first operation mode, a second target dose in the second operation mode, and a third target dose in the third operation mode, wherein the first target dose is lower than the second target dose, and the second target dose is lower than the third level of irradiation intensity.

11. The wearable electronic device (1) according to any of the preceding claims, wherein the wearable electronic device (1) is a watch configured to emit the radiation beam (11) onto a portion of a wrist of the user when the watch is worn by the user.

12. The wearable electronic device (1) according to any of the preceding claims, wherein the area of the surface of the skin of the user which receives the radiation beam (11) is between 10cm² to 60cm².

13. The wearable electronic device (1) according to any of the preceding claims, wherein the wearable electronic device (1) is configured to adjust an irradiation intensity of the radiation beam (11) and/or exposure time of the radiation beam (11) in dependence on one or more physical conditions of the user, such as heart rate, maximum rate of oxygen consumption and/or breathing characteristics of the user.

14. A system capable for providing a photobiomodulation (PBM) effect, comprising:
the wearable electronic device (1) according to any of the preceding claims; and
a second radiation device (2) configured to operate in a first state and a second state,
wherein, in the first state, the second radiation device (2) emits a second radiation beam (21) having a peak emission wavelength between 610-1400nm, and in the second state, the second radiation device (2) does not emit the second radiation beam (21), and
wherein the indication indicates whether the second radiation device (2) operates in the first state or in the second state.

15. The system according to claim 13, wherein the second radiation device (2) is configured to detect a predetermined area of the body of a user,
wherein the second radiation device (2) is configured to operate in the first state and send a deactivation signal to the wearable electronic device (1) as an indication that the second radiation source is present, when the predetermined area is detected within a defined distance; and
wherein the second radiation device (2) is configured to operate in the second state and send an activation signal to the wearable electronic device (1) as an indication that the second radiation source is absent, when the predetermined area is not detected within a defined distance.
